# EUROPEAN PATENT APPLICATION

(11) **EP 4 830 776 A2**
(43) Date of publication of application: **28.10.2026**
(21) Application number: 26173306.7
(22) Date of filing: 20.04.2026
(51) Int. Cl.: A61N 5/10, A61B 6/46

(54) **DETACHABLE DISPLAY ASSEMBLIES FOR GANTRIES**

(30) Priority: 21.04.2025 US 202519184152
(71) Applicant: Varian Medical Systems, Inc., Palo Alto, CA 94304 (US)
(72) Inventor: HANSEN, Glenn, Palo Alto, 94304 (US); SAMPSON, Jeffrey, Palo Alto, 94304 (US); BRADY, Colin, Palo Alto, 94304 (US); RODENKIRCH, Douglas, Madison, 53703 (US); GOBRECHT, Eric, Madison, 53703 (US)
(74) Representative: Mathisen & Macara LLP

(57) **Abstract**

At least one example embodiment provides a radiotherapy machine 110 including a gantry 124 and a display assembly 200 detachably coupled to the gantry 124 and rotatable with respect to the gantry 124. At least another example embodiment provides a display assembly 200 for a radiotherapy machine 110, the display assembly comprising a display 126, a swingarm 235 coupled to the display 126; mounting arms 240a, 240b coupled to the swingarm 235; and a pivotable stand 245 coupled to the display 126.

## Description

### TECHNICAL FIELD

One or more example embodiments relate to display assemblies detachably coupled to gantries such as gantries for radiotherapy machines and a radiotherapy machine comprising a gantry and display assembly according to the invention.

### BACKGROUND

Radiotherapy machines may be used in the treatment of various cancers and non-malignant conditions. Generally, ionizing radiation, including, for example, photons, e.g., X-rays, gamma rays, and charged particles, e.g., protons and electrons, is directed at an area of interest. In many cases, such ionizing radiation is generated by a linear accelerator (linac) or a cyclotron.

### SUMMARY

The scope of protection sought for various example embodiments is set out by the independent claims. The example embodiments and/or features, if any, described in this specification that do not fall under the scope of the independent claims are to be interpreted as examples useful for understanding various embodiments.

Independent of the grammatical term usage, individuals with male, female or other gender identities are included within the term.

According to at least one example embodiment, a radiotherapy machine comprises a gantry and a display assembly detachably coupled to the gantry and rotatable with respect to the gantry. The gantry may be rotatable. The display assembly may be detachably coupled to an internal portion of the gantry. The gantry may include a housing defining an aperture, and a display mount, the display assembly being detachably coupled to the display mount, the display mount including at least one first hinge to rotate the display assembly about a first axis relative to the gantry and expose the aperture. In embodiments, the display mount extends across the aperture. Suitably the display assembly includes a display screen and a swingarm including at least one second hinge configured to rotate the display screen about a second axis relative to the swingarm. The size of the aperture may correspond to the size of the display screen. The first axis and the second axis may be substantially parallel. The display assembly is suitably configured to rotate in a first direction to expose the aperture and the display screen is configured to rotate in a second direction, the first direction and the second direction being opposite directions. The display screen may be electrically couplable to the gantry. The display screen may be configured to operate when rotated about the first axis and the second axis. The display assembly may include a pivotable stand connected to the display screen. The pivotable stand may include a lever locking hinge configured to pivot the stand about multiple set positions. The swingarm may be detachable from the display mount. In embodiments the swingarm includes two mounting arms. The mounting arms may be on opposing sides of the swingarm. In embodiments, each of the mounting arms defines a slot to receive a projection of the display mount.

According to at least one example embodiment, a display assembly for a radiotherapy machine includes a display screen; a swingarm coupled to the display screen; mounting arms coupled to the swingarm; and a pivotable stand coupled to the display screen. The swingarm may include at least one hinge configured to rotate the display screen about an axis. The pivotable stand may include a lever locking hinge configured to pivot the stand about multiple set positions. The pivotable stand may include at least one first hinge, the first hinge being separate from the lever locking hinge. The pivotable stand may further includes a U-shaped bar. The mounting arms and the pivotable stand may be coupled to a rear surface of the display screen. The pivotable stand may be a single arm.

### BRIEF DESCRIPTION OF THE DRAWINGS

Example embodiments will become more fully understood from the detailed description given herein below and the accompanying drawings, wherein like elements are represented by like reference numerals, which are given by way of illustration only and thus are not limiting of this disclosure.
FIGS. 1A-1B illustrate a radiotherapy machine with a detachable display assembly in accordance with example embodiments.
FIGS. 2A-2C illustrate rotations and movements of a detachable display assembly of the radiotherapy machine of FIGS. 1A-1B, in accordance with example embodiments.
FIG. 3 illustrates a rear view of the detachable display assembly of the radiotherapy machine of FIGS. 1A-1B with a display facing away from the treatment area according to one or more example embodiments.
FIG. 4 illustrates a rear view of the detachable display assembly of the radiotherapy machine of FIGS. 1A-1B with a display facing towards the treatment area according to one or more example embodiments.
FIG. 5 illustrates the detachable display assembly of FIGS. 1A-1B being detached from a gantry according to one or more example embodiments.
FIG. 6 illustrates the detachable display assembly of FIGS. 1A-1B with a pivotable stand according to one or more example embodiments.
FIG. 7 illustrates a rear view of the display assembly of FIGS. 1A-1B with the pivotable stand according to one or more example embodiments.
FIG. 8 illustrates another pivotable stand according to one or more example embodiments.

It should be noted that these figures are intended to illustrate the general characteristics of methods, structure and/or materials utilized in certain example embodiments and to supplement the written description provided below. These drawings are not, however, to scale and may not precisely reflect the precise structural or performance characteristics of any given embodiment and should not be interpreted as defining or limiting the range of values or properties encompassed by example embodiments. The use of similar or identical reference numbers in the various drawings is intended to indicate the presence of a similar or identical element or feature.

### DETAILED DESCRIPTION

Various example embodiments will now be described more fully with reference to the accompanying drawings in which some example embodiments are shown.

Detailed illustrative embodiments are disclosed herein. However, specific structural and functional details disclosed herein are merely representative for purposes of describing example embodiments. The example embodiments may, however, be embodied in many alternate forms and should not be construed as limited to only the embodiments set forth herein.

It should be understood that there is no intent to limit example embodiments to the particular forms disclosed. On the contrary, example embodiments are to cover all modifications, equivalents, and alternatives falling within the scope of this disclosure. Like numbers refer to like elements throughout the description of the figures.

As discussed herein the terminology "one or more" and "at least one" may be used interchangeably.

It will be appreciated that a number of example embodiments may be used in combination.

When servicing radiotherapy machines, access to internal components can be very time-consuming and expensive. Components designed to reside in the center of the radiotherapy machines limit direct access to many internal components that are routinely and frequently serviced. These components may be large, heavy and must be moved out of the way for more efficient servicing procedures.

At least some example embodiments provide an improved system to allow a technician to access internal components of a radiotherapy machine. At least some example embodiments provide a display assembly that may be detachably coupled to a radiotherapy machine such that the internal components are accessible by a technician when the display assembly is rotated.

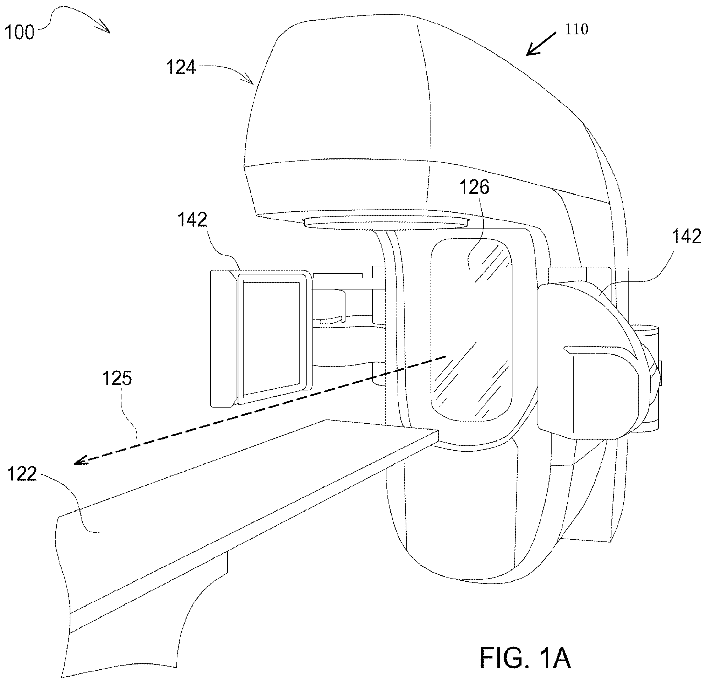

FIGS. 1A-1B illustrate a radiotherapy machine with a detachable display assembly in accordance with example embodiments.

FIG. 1A illustrates a radiotherapy system 100. The radiotherapy system 100 may be similar to the radiotherapy system depicted in FIG. 1A. The radiotherapy system may include a radiotherapy machine 110. The radiotherapy machine 110 may be in communication with a remote control system or other devices, which communicate with the radiotherapy machine 110.

The methods and systems described herein are not limited to the particular radiotherapy system described and depicted herein (e.g., radiotherapy system depicted in FIGS. 1A-1B).

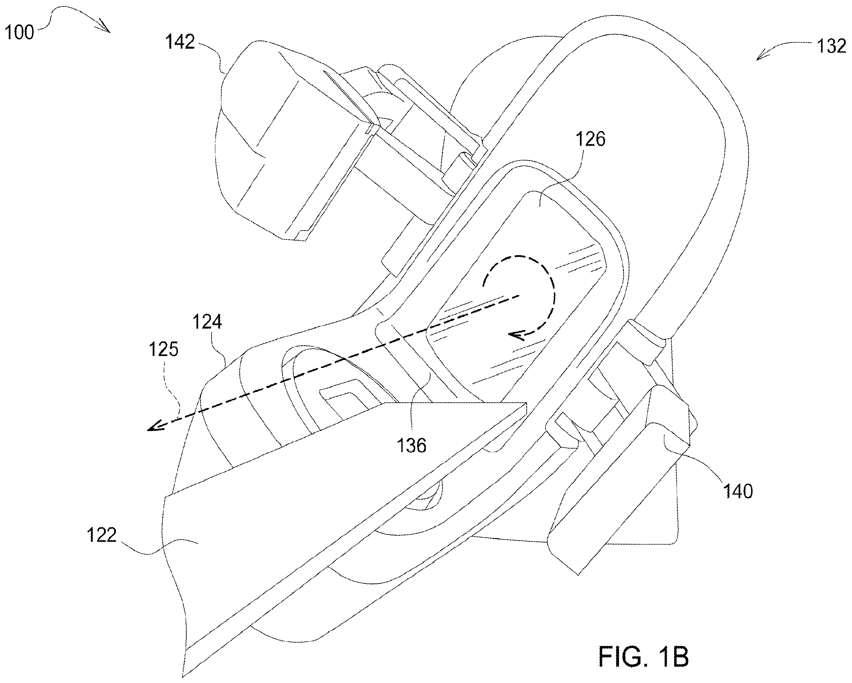

FIG. 1A, in conjunction with FIG. 1B, illustrate various features of an example radiotherapy machine 110.

The radiotherapy machine 110 may include a couch 122 where the patient is placed to receive radiation therapy treatment. The couch 122 may be configured to align the patient in a designated position during treatment. For example, patient data from the patient's RT file may be obtained, analyzed, and utilized to position the patient. In some embodiments, a doctor identifies how to align the patient on the couch 122 to optimize the treatment therapy. The couch 122 may be adjusted based on the location at which the patient will be receiving therapy. For example, if the patient's tumor is located on their left pectoral, the couch 122 may be then configured so that the radiation targets the patient's left pectoral. In some embodiments the couch 122 may be adjusted in any axial direction. For example, the couch may be elevated or lowered, it may be adjusted to move towards or away from the radiotherapy machine 110. The couch 122 can also be adjusted for rotation, pitch, and roll around an isocenter point. In other embodiments the couch may have fewer degrees of freedom/axes of movement.

The technician may manually adjust the couch 122 through the radiotherapy system 100 or the couch 122 may be automatically adjusted based on a patient's file.

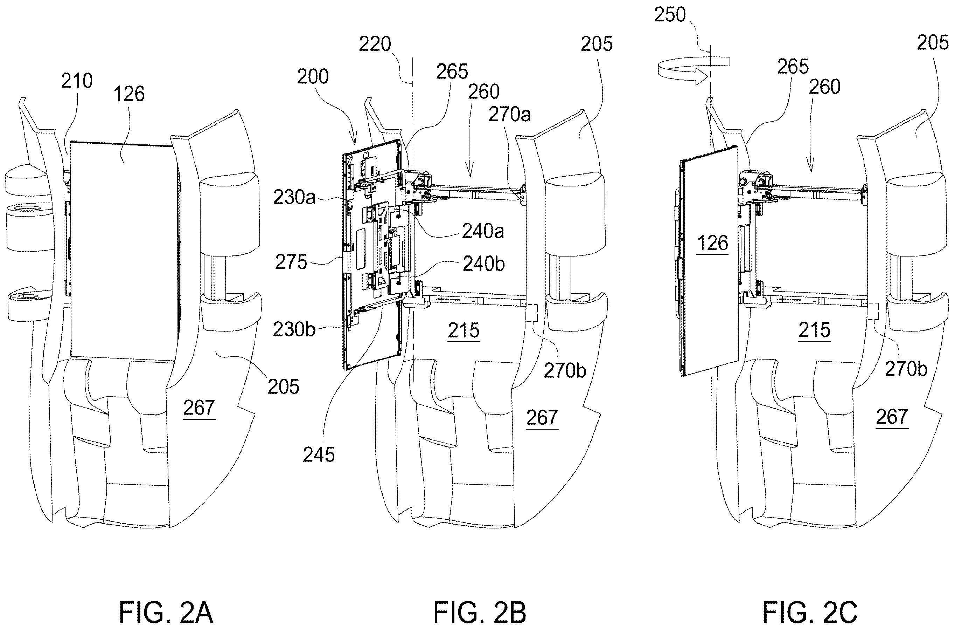

The radiotherapy machine 110 may also include a gantry 124. The gantry 124 may be disposed on a center axis 125 and is configured to rotate about the center axis 125. The gantry 124 may be positioned relative to a proximate end of the couch 122. During rotation, the gantry 124 may be configured to emit radiation towards the couch 122 while the patient is resting on the couch 122. A non-limiting example of gantry 124 in rotation is depicted in FIG. 1B. The gantry 124 may also include a display panel 126. The display panel 126 is part of a detachable display assembly 200 (e.g., shown in FIGS. 2A-4). In some configurations, the display panel 126 is configured to rotate when the gantry 124 is rotating. The rotation of the display panel 126 may be such that the display panel 126 maintains an orientation of a displayed image on the display screen relative to a person viewing the display panel 126. For example, as the gantry 124 rotates, an orientation of the content displayed to the person may be maintained.

The display panel 126 may be detachably coupled to the radiotherapy machine 110 adjacent to the proximate end of the couch 122 such that the display is in-line or approximately in-line with an external surface of the gantry 124. The display panel 126 may further be disposed on a throat of the gantry 124. The display panel 126 may be in communication with control devices to display various pages, information, and/or graphics. For instance, the display panel 126 may display a page on the display panel 126 that informs the technician of the next step/stage in the treatment process. In various other embodiments, the display panel 126 may display images regarding maintenance to be performed. For example, in some example embodiments, the display panel 126 displays a service mode application in which in-room adjustments, calibrations and other service and maintenance tools are provided.

The display panel 126 may be a touchscreen and may control the pages generated by a control device displayed on the display panel 126.

The radiotherapy system 100 may also include an imager 142 that is configured to capture medical images, such as medical images of the patient resting on the couch 122.

A distal end of the couch 122 may be diametrically opposed to the proximate end of the couch 122. The distal end of the couch 122 may also be diametrically opposed to the display panel 126.

The display panel 126 may be coupled to the gantry 124 such that the display panel 126 rotates with the gantry 124.

In some embodiments, the content of the display panel 126 may also rotate. For instance, if the gantry 124 rotates 20° in a clockwise orientation, the content of the display panel 126 may also rotate (e.g., 20° in a counterclockwise orientation). This rotation allows the patient and/or technician to easily view various pages described herein when the gantry changes its orientation. Moreover, even though the display screen is shown as having a rectangular shape, it is expressly understood that the display screen may be in other shapes (e.g., circular or oval).

FIGS. 2A-2C illustrate rotations and movements of a detachable display assembly of FIGS. 1A-1B, in accordance with example embodiments. More specifically, FIGS. 2A-2C illustrate movements of a detachable display assembly 200 that allow access to an internal portion of a gantry in the throat of the gantry. This access allows a service person to access internal components of the radiotherapy machine.

The display assembly 200 includes the display panel 126, latching arms 230a, 230b, a swingarm 235, mounting arms 240a, 240b and a pivotable stand 245.

FIG. 2A shows the display panel 126 in a position such that the radiotherapy machine may apply treatment to a patient without interrupting the patient couch 122. The gantry 124 includes a housing 205 at a throat portion of the gantry 124. The housing 205 defines an aperture 210. The aperture 210 provides access to an internal area 215 that includes internal components of the radiotherapy machine, e.g., for maintenance. As will be described in greater detail, the display panel 126 may be rotated into the aperture 210 to cover the internal area 215 and prevent access to the internal components. This allows easy access to the internal components when maintenance is performed while also providing a manner to cover the internal area 215.

The aperture 210 may have a size that corresponds to the display panel 126. In some example embodiments, the aperture 210 may be sized such that the display panel 126 is flush or approximately flush with an external surface 267 of the housing 205. In other example embodiments, the aperture 210 may be slightly larger than the display panel 126 such that a service person may access a gap between the display panel 126 and the housing 205 when the display panel 126 is in the aperture 210. In some example embodiments, the gap provides an area for the service person to access levers to release a side of the display assembly 200 from the gantry. A cover (not shown) may be placed over the gap such that the display panel 126 becomes flush or approximately flush with the external surface 267 of the housing 205 when the display assembly 200 is locked to the housing 205 (described in further detail below).

As shown in FIG. 2B, the display assembly 200 is rotatable with respect to the gantry about a first axis 220 to expose the internal area 215. The first axis 220 extends in a longitudinal direction of the housing 205 (throat portion). The display assembly 200 may rotate in a clockwise direction about the axis 220 to expose the internal area 215 and permit access.

In addition, as shown in FIG. 2C, the display panel 126 is rotatable about a second axis 250 relative to the swingarm 235. More specifically, the display panel 126 is rotatable in the counterclockwise direction about the axis 250. In various other embodiments, the display panel 126 may display images regarding maintenance to be performed which permits the display panel 126 to be viewed by a service person while performing maintenance on the radiotherapy machine 110. By rotating the display panel 126 in the counterclockwise direction about the axis 250, the technician can view the content displayed on the display panel 126 while performing maintenance on the internal components. The axis 250 is parallel or substantially parallel to the axis 220.

Thus, the display assembly 200 is configured to rotate in a first direction to expose the aperture 210 and the display panel 126 is configured to rotate in a second direction with the first direction and the second direction being opposite directions.

A display mount 260 is located in the internal area 215 of the housing 205. The housing 205 may include an internal surface 265 and the external surface 267. The display mount 260 may be connected to the internal surface 265 and extend across the aperture 210. In some example embodiments, the display mount 260 extends along a direction that is normal to the longitudinal axis of the housing 205. In the example embodiment shown in FIGS. 2A-2C, a width of the aperture 210 is the direction that is normal to the longitudinal axis of the housing 205. Thus, the display mount 260 may be coupled on opposing side of the housing 205 that define the aperture 210.

The display assembly 200 is detachably coupled to the display mount 260. The display assembly includes couplers 270a and 270b that couple a first side 275 of the display assembly 200 to the housing 205. More specifically, the latching arms 230a, 230b of the display assembly 200 are on the first side of the display assembly 200. When the display panel 126 is in the position shown in FIG. 2A, the latching arms 230a, 230b may be coupled to the couplers 270a, 270b, respectively, to maintain the display panel 126 in the position shown in FIG. 2A. In some embodiments, the latching arms 230a, 230b may be coupled to the couplers 270a, 270b using screws, latches, captive fasteners and/or non-captive fasteners to secure and release the display panel 126 from the couplers 270a, 270b.

When the couplers 270a, 270b release the display assembly 200 and/or no longer couple the display assembly 200 to the housing 205 (i.e., the couplers 270a, 270b are not coupled to the latching arms 230a, 230b, respectively), the display assembly 200 may rotate about the axis 220.

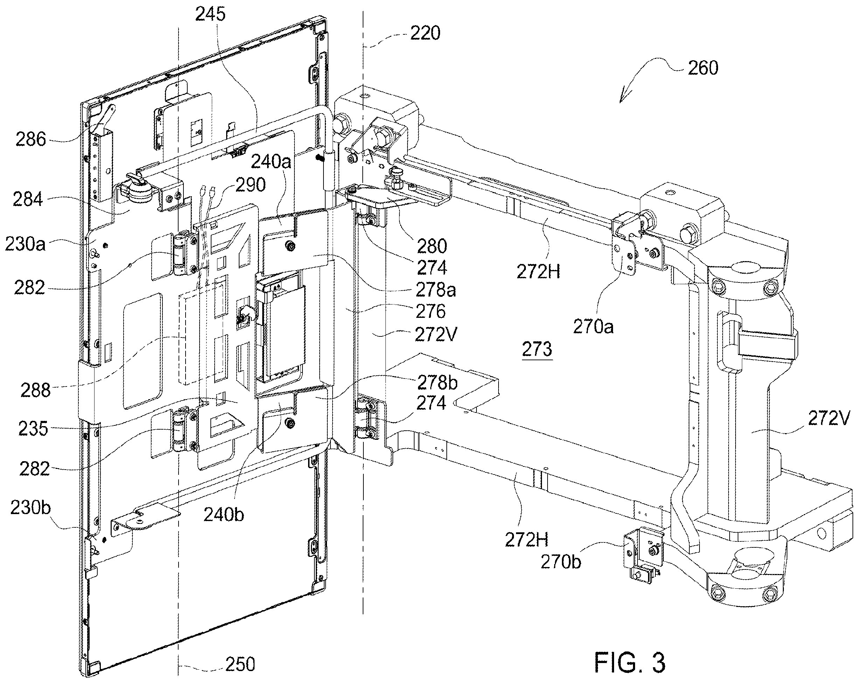

FIG. 3 illustrates a rear view of the display assembly of FIGS. 1A-1B with a display facing away from the treatment area according to one or more example embodiments.

The display mount 260 includes two opposing vertical supports 272V and two horizontal opposing crossbars 272H that form a rectangular shape defining an internal area 273. The internal area 273 is part of the internal area 215. One of the opposing vertical supports 272V includes two hinges 274 mounted on opposing ends of the vertical support 272V. The hinges 274 allow the display assembly 200 to rotate about the axis 220. While two hinges 274 are illustrated, it should be understood the one hinge or more than two hinges may be used.

The display mount 260 further includes a swinging bar 276 coupled to the hinges 274. The swinging bar 276 includes mounting tabs 278a, 278b at opposing ends of the swinging bar 276. A follower arm 280 limits rotation of the display panel 126, preventing the display panel 126 from extending beyond the rotation limit and interfering with the covers and other components. The follower arm 280 may also include a hole in which a spring-loaded locking pin plunges into the hole to lock the rotation at this limit. The lock pin may be pulled to unlock the follower arm 280 before rotating the display panel 126 back into the aperture 210.

The mounting tabs 278a, 278b are detachably coupled to the mounting arms 240a, 240b, respectively. In an example embodiment, the mounting tabs 278a, 278b include internal horizontal projections and the mounting arms 240a, 240b includes slots to receive the projections such that the mounting arms 240a, 240b "sit" on the mounting tabs 278a, 278b, respectively.

The mounting arms 240a, 240b are part of the swingarm 235. The swingarm 235 further includes two hinges 282 that permit the display panel 126 to rotate about the axis 250. The hinges 282 are on opposing ends of a first side of the swingarm 235 and the mounting arms 240a, 240b are on opposing ends of the second side of the swingarm 235. The first and second sides of the swingarm 235 extend in a longitudinal direction of the swingarm 235 and a longitudinal direction of the display panel 126. The hinges 282 are coupled to a plate 284 which is coupled to a rear side 286 of the display panel 126. The rear side 286 of the display panel 126 opposes a front side of the display panel 126 that displays the content. The remaining portion of the swingarm 235, including the mounting arms 240a, 240b are not directly coupled to the plate 284.

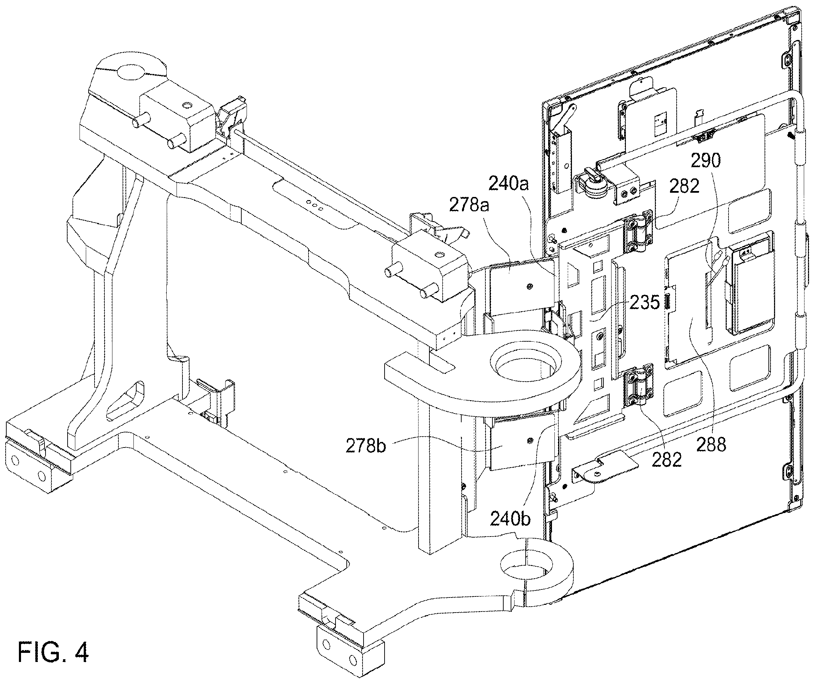

Thus, the display panel 126 may rotate about the axis 250 (via the hinges 282) while still being coupled to the display mount 260 via the mounting arms 240a, 240b being coupled to the mounting tabs 278a, 278b, as shown in FIG. 4.

As shown in FIGS. 3 and 4, the display assembly 200 further includes an electrical panel 288 mounted to the rear side 286 of the display panel 126. The electrical panel 288 provides ports for various connection cables 290 (e.g., power and display such as HDMI). The connection cable 290 are detachable to corresponding cables in the gantry 124. For example, the power cable of the display assembly 200 is detachably connected to a power cable in the gantry 124. This permits the display panel 126 to be electrically couplable to the gantry 124 (and the radiotherapy system 100) and to operate when rotated about the axis 220 and the axis 250.

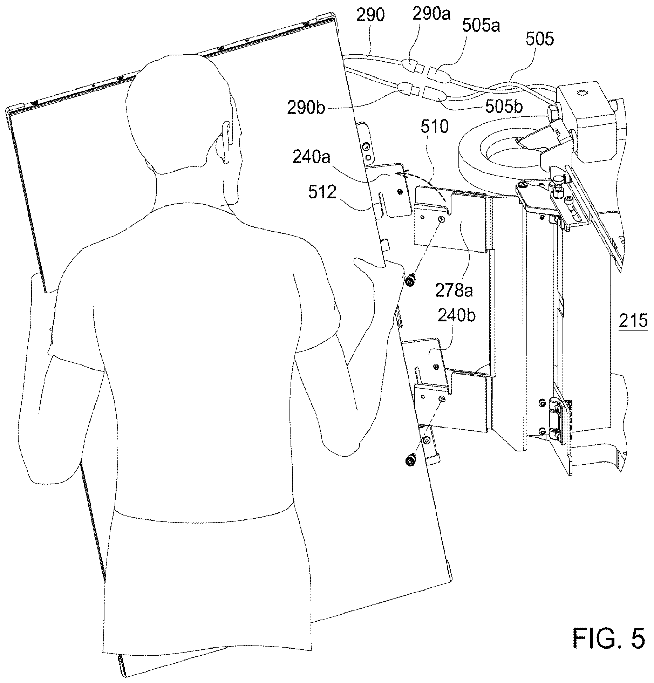

FIG. 5 illustrates an example embodiment of detaching the display assembly 200 from the display mount 260. In FIG. 5, the display assembly 200 may be detached from the display mount 260 by disconnecting cables 290 from corresponding cables 505 of the gantry (e.g., power and video cables). More specifically, each of the two cables 505 (e.g., one for video and another for power) 290 has a connector 290a, 290b. Similarly, each of the two cables 505 has a connector 505a, 505b. In some example embodiments, the connector 290a may be disconnected from the connector 505a by pulling the two connectors apart. Similarly, the connector 290b may be disconnected from the connector 505b by pulling the two connectors apart. In some embodiments, connectors 505a and 505b may be connected directly to the to the electrical panel 288 on rear side 286.

Additionally, the mounting arm 240a may be pulled out of the mounting tab 278a. More specifically, the mounting arm 240a includes a slot 512 that sits on an internal portion of the tab 278a. A technician may remove the display assembly 200 from the display mount 260 by lifting and pulling the display assembly 200 in the direction indicated by arrow 510. While not fully illustrated, the mounting arm 240b become decoupled from the tab 278b in a similar manner and simultaneously with the decoupling of the mounting arm 240a from the tab 278a.

When the display assembly 200 is detached from the gantry 124, the pivotable stand 245 may be used to stand the display panel 126 on a surface at an angle. The display panel 126 may be placed on the surface at an angle such that a technician may view the display panel 126 at a desirable angle.

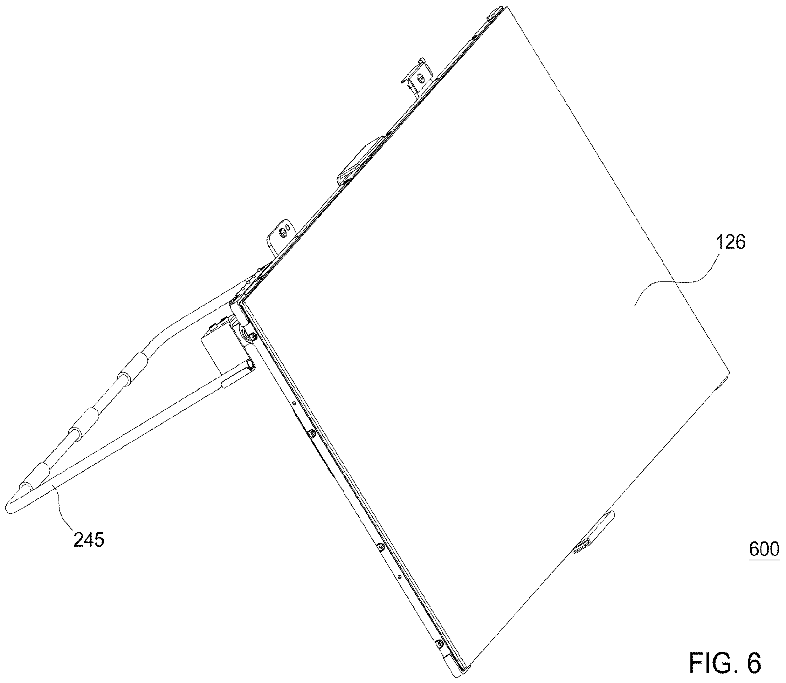

FIG. 6 illustrates the display assembly 200 being detached from the gantry 124 according to one or more example embodiments. As shown in FIG. 6, the pivotable stand 245 is pivoted at a desired viewing angle on a surface 600. In FIG. 6, the surface 600 is shown as a floor of a room having the radiotherapy machine. However, example embodiments are not limited thereto, and the display assembly may be placed on another flat surface such as a table. Because the display assembly 200 includes cables 290, the display assembly may be configured to be connected to separate cables 610 that also provide power and video from the radiotherapy machine 110.

In other embodiments, the cables 610 may connect directly to the rear of the display panel 126 for HDMI and Power. Additionally or alternatively, an HDBaseT receiver may be connected to the same connectors on the back side of the display panel 126 (via power and HDMI cables) and a power and CAT6 cable will connect between the HDBaseT receiver and the radiotherapy machine.

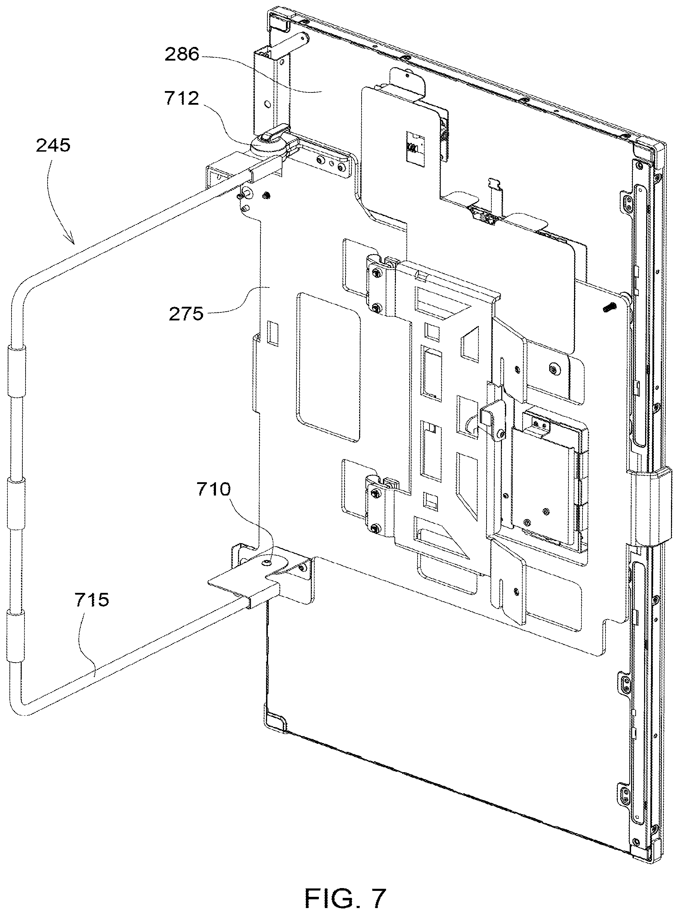

FIG. 7 illustrates a rear view of the display assembly of FIGS. 1A-1B with the pivotable stand according to one or more example embodiments.

As shown in FIG. 7, the pivotable stand 245 is connected to the rear side 286 of the display panel 126 and includes at least one lever locking hinge 710, another hinge 712 and a U-shaped bar 715. Ends of the U-shaped bar are connected to the hinges 710, 712, respectively. The hinges are located on the side 275 of the display assembly 200.

The lever locking hinge 710 is configured to pivot the U-shaped bar 715 about multiple set positions. In some example embodiments, the hinge 712 is also a lever locking hinge. However, in other example embodiments, the hinge 712 is another type of hinge or is a component that permits the U-shaped bar to pivot about the location of the hinge 712. The U-shaped bar 715 rotates about an axis that is along a longitudinal axis of the display panel 126. However, example embodiments are not limited thereto.

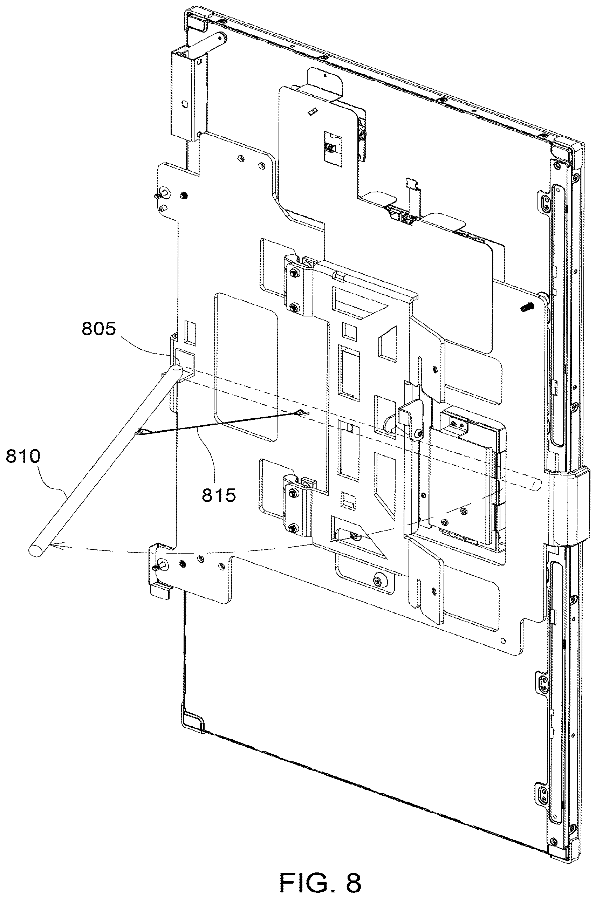

While a U-shaped bar 715 is illustrated in FIG. 7, other shapes may be used to stand the display panel 126 up at an angle relative to a base surface (e.g., surface of a table, surface of a floor of a room) such as shown in FIG. 8.

In FIG. 8, a pivotable stand may include a pivot support 805, a rod (leg) 810 and a chain 815.

Although the terms first, second, etc. may be used herein to describe various elements, these elements should not be limited by these terms. These terms are only used to distinguish one element from another. For example, a first element could be termed a second element, and similarly, a second element could be termed a first element, without departing from the scope of this disclosure. As used herein, the term "and/or," includes any and all combinations of one or more of the associated listed items.

When an element is referred to as being "connected," or "coupled," to another element, it can be directly connected or coupled to the other element or intervening elements may be present. By contrast, when an element is referred to as being "directly connected," or "directly coupled," to another element, there are no intervening elements present. Other words used to describe the relationship between elements should be interpreted in a like fashion (e.g., "between," versus "directly between," "adjacent," versus "directly adjacent," etc.).

The terminology used herein is for the purpose of describing particular embodiments only and is not intended to be limiting. As used herein, the singular forms "a," "an," and "the," are intended to include the plural forms as well, unless the context clearly indicates otherwise. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

Specific details are provided in the following description to provide a thorough understanding of example embodiments. However, it will be understood by one of ordinary skill in the art that example embodiments may be practiced without these specific details. For example, systems may be shown in block diagrams so as not to obscure the example embodiments in unnecessary detail. In other instances, well-known processes, structures and techniques may be shown without unnecessary detail in order to avoid obscuring example embodiments.

As discussed herein, illustrative embodiments are described with reference to acts and symbolic representations of operations (e.g., in the form of flow charts, flow diagrams, data flow diagrams, structure diagrams, block diagrams, etc.) that may be implemented as program modules or functional processes include routines, programs, objects, components, data structures, etc., that perform particular tasks or implement particular abstract data types and may be implemented using existing hardware, for example, processing or control circuitry such as, but not limited to, one or more processors, one or more Central Processing Units (CPUs), one or more controllers, one or more arithmetic logic units (ALUs), one or more digital signal processors (DSPs), one or more microcomputers, one or more field programmable gate arrays (FPGAs), one or more System-on-Chips (SoCs), one or more programmable logic units (PLUs), one or more microprocessors, one or more Application Specific Integrated Circuits (ASICs), or any other device or devices capable of responding to and executing instructions in a defined manner.

Although a flow chart may describe the operations as a sequential process, many of the operations may be performed in parallel, concurrently or simultaneously. In addition, the order of the operations may be re-arranged. A process may be terminated when its operations are completed, but may also have additional steps not included in the figure. A process may correspond to a method, function, procedure, subroutine, subprogram, etc. When a process corresponds to a function, its termination may correspond to a return of the function to the calling function or the main function.

As disclosed herein, the term "memory," "storage medium," "processor readable medium," "computer readable storage medium" or "non-transitory computer readable storage medium" may represent one or more devices for storing data, including read only memory (ROM), random access memory (RAM), magnetic RAM, core memory, magnetic disk storage mediums, optical storage mediums, flash memory devices and/or other tangible machine-readable mediums for storing information. The term "computer-readable medium" may include, but is not limited to, portable or fixed storage devices, optical storage devices, and various other mediums capable of storing, containing or carrying instruction(s) and/or data.

Furthermore, example embodiments may be implemented by hardware, software, firmware, middleware, microcode, hardware description languages, or any combination thereof. When implemented in software, firmware, middleware or microcode, the program code or code segments to perform the necessary tasks may be stored in a machine or computer readable medium such as a computer readable storage medium. When implemented in software, a processor or processors will perform the necessary tasks. For example, as mentioned above, according to one or more example embodiments, at least one memory may include or store computer program code, and the at least one memory and the computer program code may be configured to, with at least one processor, cause a network element or network device to perform the necessary tasks. Additionally, the processor, memory and example algorithms, encoded as computer program code, serve as means for providing or causing performance of operations discussed herein.

The terms "including" and/or "having," as used herein, are defined as comprising (i.e., open language). The term "coupled," as used herein, is defined as connected, although not necessarily directly, and not necessarily mechanically. Terminology derived from the word "indicating" (e.g., "indicates" and "indication") is intended to encompass all the various techniques available for communicating or referencing the object/information being indicated. Some, but not all, examples of techniques available for communicating or referencing the object/information being indicated include the conveyance of the object/information being indicated, the conveyance of an identifier of the object/information being indicated, the conveyance of information used to generate the object/information being indicated, the conveyance of some part or portion of the object/information being indicated, the conveyance of some derivation of the object/information being indicated, and the conveyance of some symbol representing the object/information being indicated.

Benefits, other advantages, and solutions to problems have been described above with regard to specific embodiments. However, the benefits, advantages, solutions to problems, and any element(s) that may cause or result in such benefits, advantages, or solutions, or cause such benefits, advantages, or solutions to become more pronounced are not to be construed as a critical, required, or essential feature or element of any or all the claims.

### Non-Limiting Illustrative Embodiments

Illustrative embodiment 1. A radiotherapy machine comprising: a gantry; and a display assembly detachably coupled to the gantry and rotatable with respect to the gantry.

Illustrative embodiment 2. The radiotherapy machine of Illustrative embodiment 1, wherein the display assembly is detachably coupled to an internal portion of the gantry.

Illustrative embodiment 3. The radiotherapy machine of any one of Illustrative embodiments 1-2, wherein the gantry includes, a housing defining an aperture, and a display mount, the display assembly being detachably coupled to the display mount, the display mount including at least one first hinge to rotate the display assembly about a first axis relative to the gantry and expose the aperture.

Illustrative embodiment 4. The radiotherapy machine of Illustrative embodiment 3, wherein the display assembly includes, a display screen, and a swingarm including at least one second hinge configured to rotate the display screen about a second axis relative to the swingarm.

Illustrative embodiment 5. The radiotherapy machine of Illustrative embodiment 4, wherein the first axis and the second axis are substantially parallel, and the display assembly is configured to rotate in a first direction to expose the aperture and the display screen is configured to rotate in a second direction, the first direction and the second direction being opposite directions.

Illustrative embodiment 6. The radiotherapy machine of Illustrative embodiment 4, wherein the display screen is electrically couplable to the gantry and the display screen is configured to operate when rotated about the first axis and the second axis.

Illustrative embodiment 7. The radiotherapy machine of any one of Illustrative embodiments 4-6, wherein the display assembly includes, a pivotable stand connected to the display screen.

Illustrative embodiment 8. The radiotherapy machine of Illustrative embodiment 7, wherein the pivotable stand includes, a lever locking hinge configured to pivot the stand about multiple set positions.

Illustrative embodiment 9. The radiotherapy machine of any one of Illustrative embodiments 4-8, wherein the swingarm is detachable from the display mount.

Illustrative embodiment 10. The radiotherapy machine of Illustrative embodiment 9, wherein the swingarm includes two mounting arms, the mounting arms being on opposing sides of the swingarm, each of the mounting arms defining a slot to receive a projection of the display mount.

Illustrative embodiment 11. The radiotherapy machine of any one of Illustrative embodiments 3-10, wherein the display mount extends across the aperture.

Illustrative embodiment 12. The radiotherapy machine of any one of Illustrative embodiments 4-11, wherein a size of the aperture corresponds to a size of the display screen.

Illustrative embodiment 13. The radiotherapy machine of any one of Illustrative embodiments 1-12, wherein the gantry is rotatable.

Illustrative embodiment 14. A display assembly for a radiotherapy machine, the display assembly comprising: a display screen; a swingarm coupled to the display screen; mounting arms coupled to the swingarm; and a pivotable stand coupled to the display screen.

Illustrative embodiment 15. The display assembly of Illustrative embodiment 14, wherein the swingarm includes at least one hinge configured to rotate the display screen about an axis.

Illustrative embodiment 16. The display assembly of any one of Illustrative embodiments 14-15, wherein the pivotable stand includes, a lever locking hinge configured to pivot the stand about multiple set positions.

Illustrative embodiment 17. The display assembly of Illustrative embodiment 16, wherein the pivotable stand includes at least one first hinge, the first hinge being separate from the lever locking hinge.

Illustrative embodiment 18. The display assembly of any one of Illustrative embodiments 16-17, wherein the pivotable stand further includes a U-shaped bar.

Illustrative embodiment 19. The display assembly of any one of Illustrative embodiments 14-18, wherein the swingarm, the mounting arms and the pivotable stand are coupled to a rear surface of the display screen.

Illustrative embodiment 20. The display assembly of Illustrative embodiment 14, wherein the pivotable stand is a single arm.

## Claims

1. A radiotherapy machine (110) comprising:
a gantry (124); and
a display assembly (200) detachably coupled to the gantry and rotatable with respect to the gantry

2. The radiotherapy machine of claim 1, wherein the display assembly (200) is detachably coupled to an internal portion of the gantry (124) and optionally the gantry (124) includes,
a housing (205) defining an aperture (210), and
a display mount (260), the display assembly (200) being detachably coupled to the display mount (260), the display mount including at least one first hinge (274) to rotate the display assembly (200) about a first axis (220) relative to the gantry (124) and expose the aperture (210).

3. The radiotherapy machine of claim 1 or 2, wherein the display assembly (200) includes,
a display screen (126), and
a swingarm (235) including at least one second hinge (282) configured to rotate the display screen about a second axis (250) relative to the swingarm and, optionally,
the first axis (220) and the second axis (250) are substantially parallel, and the display assembly (200) is configured to rotate in a first direction to expose the aperture (210) and the display screen (126) is configured to rotate in a second direction, the first direction and the second direction being opposite directions and optionally the display assembly (200) includes,
a pivotable stand (245) connected to the display screen.

4. The radiotherapy machine of claim 3, wherein the display screen (126) is electrically couplable to the gantry (124) and the display screen is configured to operate when rotated about the first axis (220) and the second axis (250).

5. The radiotherapy machine of claim 3 or 4, wherein the pivotable stand (245) includes,
a lever locking hinge (710) configured to pivot the stand about multiple set positions.

6. The radiotherapy machine of claim 3, 4 or 5, wherein the swingarm (235) is detachable from the display mount (260) and, optionally, the swingarm (235) includes two mounting arms (240a, 240b), the mounting arms being on opposing sides of the swingarm (235), each of the mounting arms defining a slot (512) to receive a projection of the display mount (260).

7. The radiotherapy machine of any one of claims 3 to 6, wherein a size of the aperture (210) corresponds to a size of the display screen (126).

8. The radiotherapy machine of any one of claims 2-7, wherein the display mount (260) extends across the aperture (210).

9. The radiotherapy machine of any one of the preceding claims, wherein the gantry (124) is rotatable.

10. A display assembly (260) for a radiotherapy machine (110), the display assembly comprising:
a display screen (126);
a swingarm (235) coupled to the display screen;
mounting arms (240a, 240b) coupled to the swingarm; and
a pivotable stand (245) coupled to the display screen (126).

11. The display assembly of claim 10, wherein the swingarm (235) includes at least one hinge (274) configured to rotate the display screen (126) about an axis and, optionally, the pivotable stand (245) includes,
a lever locking hinge (710) configured to pivot the stand (245) about multiple set positions.

12. The display assembly of claim 10 or 11, wherein the pivotable stand (245) includes at least one first hinge (712), the first hinge (712) being separate from the lever locking hinge (710).

13. The display assembly of any one of claims 10 to 12, wherein the pivotable stand (245) further includes a U-shaped bar (715).

14. The display assembly of any one of claims 10-13, wherein the swingarm (235), the mounting arms (240a, 240b) and the pivotable stand (245) are coupled to a rear surface (286) of the display screen (126).

15. The display assembly of any one of claims 10 to 14, wherein the pivotable stand (245) is a single arm.
